# EUROPÄISCHE PATENTSCHRIFT

(11) **EP 4 526 675 B1**
(45) Veröffentlichungstag und Bekanntmachung des Hinweises auf die Patenterteilung: **06.08.2025**
(21) Anmeldenummer: 23836341.0
(22) Anmeldetag: 17.11.2023
(51) Int. Cl.: G01N 31/22, G01N 21/77, G01N 21/78

(54) **INDIKATORETIKETT ZUR OPTISCHEN ERMITTLUNG DER KONZENTRATION EINES ANALYTEN IN EINEM FLUIDGEMISCH**
INDICATOR LABEL FOR THE OPTICAL DETERMINATION OF THE CONCENTRATION OF AN ANALYTE IN A FLUID MIXTURE
ETIQUETTE INDICATRICE POUR LA DETERMINATION OPTIQUE DE LA CONCENTRATION D'UN ANALYTE DANS UN MELANGE DE FLUIDES

(30) Priorität: 25.11.2022 DE 102022131238
(43) Veröffentlichungstag der Anmeldung: 26.03.2025
(73) Patentinhaber: Wannenwetsch, Alexander, 97647 Willmars (DE)
(72) Erfinder: Wannenwetsch, Alexander, 97647 Willmars (DE)
(74) Vertreter: Scharfenberger, Burkhard
(86) Internationale Anmeldenummer: PCT/DE2023/100889
(87) Internationale Veröffentlichungsnummer: WO 2024/109988

(56) Entgegenhaltungen:
- EP-A1- 3 893 230
- EP-A1- 3 974 486
- WO-A1-97/31599
- JP-A- 2000 055 902
- JP-A- 2006 044 776

## Beschreibung

Vorliegende Erfindung betrifft eine Verpackung für eine Lebensmittel oder ein anderes Verpackungsgut gemäß dem Oberbegriff des Anspruchs 1.

Bei Lebensmitteln und anderen empfindlichen Gütern ist es für eine möglichst lange Haltbarkeit wichtig, diese gut zu verpacken. Die Verpackung sollte nach der Versiegelung in jedem Fall Keim und Schmutzdicht sein. Um eine weitere Erhöhung der Haltbarkeit zu erreichen, werden darüber hinaus auch gasdichte Verpackungen verwendet, bei denen, eine korrekte Versiegelung vorausgesetzt, nahezu kein Gasaustausch zwischen der Atmosphäre in der Verpackung und der äußeren Atmosphäre stattfindet. Hierfür werden Verpackungen aus porenfreien Materialen eingesetzt, welche zudem idealerweise auch nur einen geringen diffusionsbedingten Gasaustausch erlauben.

Bevorzugt werden insbesondere Verpackungen aus Kunststofffolie, etwa in Form eines Schlauchbeutels, oder zweiteilige Verpackungen aus einer Unterschale, welche aus Kunststoff oder auch aus beschichteten Karton bestehen kann, und eine diese verschließende Folie, beispielsweise eine transparente Kunststofffolie. Zu einer weiteren Herabsetzung der Teilchendiffusion durch die Verpackung kann auch eine Metallfolie oder eine metallbeschichtete Kunststofffolie eingesetzt werden. Derartige gasdichte Verpackungen erlauben es, im Inneren der Verpackung eine relativ zur normalen Umgebungsluft modifizierte Atmosphäre einzusetzen, weswegen Sie auch als MAP (Modified Atmosphere Packaging) Industrieverpackungen bezeichnet werden.

Die so modifizierte Atmosphäre besteht üblicherweise aus einem oder mehreren Inertgasen. Vorwiegend wird hier aus wirtschaftlichkeitsgründen Stickstoff eingesetzt. Inertgase werden deshalb verwendet, um die Oxidation des Lebensmittels oder anderen Gut durch den reaktiven Luftsauerstoff zu verhindern. Es ist also das Ziel einer üblichen MAP Verpackung, nach der Versiegelung eine möglichst geringen Restsauerstoffgehalt in der Verpackung zu finden.

Diese modifizierte Atmosphäre soll die versiegelte Verpackung möglichst lange halten. Die Schwachstelle der Verpackung ist hierbei weniger das Verpackungsmaterial, selbst wenn dieses eine gewisse diffusionsbedingte Durchlässigkeit besitzt, sondern die Versiegelung. Bei den üblicherweise maschinell vorgenommenen Versiegelungsvorgängen kann es aus verschiedenen Gründen vorkommen, dass die Versiegelung nur unvollständig ist. Zum einen kann die Verpackung beim Versiegeln nicht genau in der korrekten Position liegen, sodass der Versiegelungsstempel die Verpackung in manchen Bereichen nur unvollständig oder gar nicht versiegelt, oder es können sich Stücke des Verpackungsguts zwischen den zu versiegelnden Teilen Verpackung befinden und die Versiegelung kompromittieren.

Um sicher zu stellen, dass die produzierten und ausgelieferten Lebensmittel das aufgedruckte Mindesthaltbarkeitsdatum auch erreichen können, ist es daher bekannt, unmittelbar im Anschluss an die Versiegelung die Dichtheit der Verpackung zu testen. Dafür wird die Verpackung mechanischen Kräften oder sich änderndem Druck ausgesetzt, wodurch im Falle einer fehlerhaften Versiegelung eine schnelle Änderung der modifizierten Atmosphäre innerhalb der Verpackung erreicht wird. In einem anschließenden Schritt wird dann die Zusammensetzung der Atmosphäre gemessen und bei Abweichung vom erwarteten bzw. zuvor gemessenen Wert die Verpackung aussortiert. Verpackungen, die Gasindikatoren enthalten sind aus EP3974486A1 und JP2006044776 bekannt.

Das Überprüfen der Atmosphäre in der Verpackung kann dabei zerstörerisch erfolgen, beispielsweise durch Einstechen in die Verpackung mit einer Messsonde oder -lanze, mit welcher eine Probe der Atmosphäre entnommen wird. Es sind im Stand der Technik jedoch auch nicht zerstörerische Verfahren bekannt.

Bei Verfahren, bei dem die Zusammensetzung der Atmosphäre insbesondere der vorhandene Restsauerstoff, mit optischen Mitteln bestimmt wird, befindet sich im Inneren der Verpackung, beispielsweise auf der Innenseite der Verpackungsfolie aufgedruckt, ein fluoreszenter Farbstoff, der von außen mit Licht der passenden Frequenz angeregt und dessen Emissionsantwort im Zeitverlauf gemessen wird. Aus den so bestimmten Parametern der Fluoreszenz, insbesondere der Emissionsintensität und/oder der Abklingzeit nach Abschalten der Anregung, kann dann, eine vorherige korrekte Kalibrierung vorausgesetzt, auf die Sauerstoffkonzentration geschlossen werden. Hierbei ist es bei den üblicherweise verwendeten Porphyrinfarbstoffen insbesondere so, dass sich unter Anwesenheit von Sauerstoff zum einen die Abklingzeit verkürzt, zum anderen auch die Intensität der Emissionsantwort vermindert. Dieser Effekt wird als sauerstoffinduziertes Quenching der Fluoreszenz bezeichnet. Ein solches Verfahren wird beispielsweise in der internationalen Veröffentlichungsschrift WO 2018/202784 A1 beschrieben.

In einer weiteren Offenlegungsschrift einer internationalen Patentanmeldung, der WO 2017/125386 A2, wird eine Schlauchbeutelverpackungsmaschine vorgestellt, welche über Mittel verfügt, um in transparenten Bereichen der Verpackung Sensorpunkte aufzudrucken, mit denen danach dem Versiegeln des Schlauchbeutels der Restsauerstoffgehalt auf die vorbeschriebene Art optisch bestimmt werden kann. Da die Messung an den sich vor dem Sensor vorbeibewegenden Schlauchbeuteln vorgenommen wird, schlägt diese Schrift vor, die Sensorpunkte länglich auszugestalten, wobei die Längsrichtung entlang der Bewegungsrichtung des Schlauchbeutels orientiert ist, wodurch der optische Sensor mehr Zeit zur korrekten Erfassung der Sensorpunkte erhält.

In der internationalen Veröffentlichungsschrift WO 2018/011307 A1 wird eine Verpackungsmaschine und ein Verfahren zum Verpacken von Lebensmitteln in zweiteiligen Verpackungen aus rasterartig angeordneten schalenförmigen Unterteilen, welche durch eine aufgesiegelte transparente Folie verschlossen werden, vorgestellt. Hierbei ist an der Innenseite jeder Verpackung auf der Unterseite der Folie ein einen fluoreszierenden Farbstoff enthaltender Sensorpunkt aufgedruckt. Ein optischer Sensor der Verpackungsmaschine bestimmt nach dem Versiegeln und ggf. einer Dichtigkeitsprüfung den Restsauerstoffgehalt der Verpackung. Anhand der gemessenen Wertes wird für jede Verpackung ein individuelles Mindesthaltbarkeitsdatum errechnet und in ein auf der Außenseite aufgeklebtes RFID Etikett eingespeichert

Die veröffentlichte europäische Patentanmeldung EP 0449798 A2 stellt ein Verfahren zur Qualitätskontrolle von verpackten, organischen Stoffen vor, bei dem ein flächenförmiges optisches Sensorelement in die Verpackung eingefügt wird, wobei das Sensorelement mit dem Lebensmittel in Kontakt steht und die Änderung der Gaszusammensetzung im Innenraum der Verpackung ermitteln kann, indem für verschiedene Analyte sensitive chemische Stoffe in den Sensorelement enthalten sind, und die Änderung der Konzentration der jeweiligen Analyten, bei denen es sich um Abbauprodukte der verpackten Lebensmittel handelt, optisch ermittelbar ist. Das Sensorelement besteht aus einer Indikatorschicht, welche in Richtung des Lebensmittels von einer hydrophoben Polymerschicht abgedeckt ist. Es ist mit der der Polymerschicht gegenüber liegenden Seite an die Innenseite der Verpackung angeheftet. Der Nachteil des hier vorgeschlagenen Sensorelements ist seine vergleichsweise große räumliche Ausdehnung, sowie die Tatsache, dass keine Maßnahmen getroffen werden, die Indikatorschicht so zu gestalten, dass ein zuverlässiges Messsignal erfasst werden kann. Zum anderen ist das Sensorelement vergleichsweise voluminös und großflächig und reduziert so den im Inneren der Verpackung vorhandenen, für das eigentliche Verpackungsgut zur Verfügung stehenden Raum. Zudem ist der durch ein solch großes Sensorelement verursachte Ressourcenverbrauch bei einem Masseneinsatztäglich alleine werden in Deutschland hunderttausende MAP-Verpackungen hergestellt - nicht sehr vorteilhaft.

Die Veröffentlichungsschrift WO 2022/055433 A1 stellt einen Sauerstoffsensor und ein diesen enthaltendes Sauerstoffsensorfilmetikett vor. Der Sauerstoffsensor umfasst einen mehrschichtigen Polyelektrolytfilm und einen farbstoffträger ein Fluoreszenzfarbstoff. Das Sauerstoffsensoretikett umfasst einen solchen Sauerstoffsensor, der weiterhin mit einer Lebensmittelschicht und einer darüber liegenden Schutzschicht überdeckt ist, und sieht weiterhin auf der Unterseite des Sauerstoffsensors bzw. der Sauerstoffsensorschicht eine Haftschicht zum Befestigen des Sensoretiketts an einer Oberfläche einer Verpackung vor. Der Nachteil dieses Etiketts liegt in seinem vergleichsweise komplexen Aufbau und damit hohen Herstellungskosten. Weiterhin hat es eine relativ große Fläche, sodass, um eine korrekte optische Auslesung zu gewährleisten, einen entsprechend großer transparenter Bereich in der Verpackung vorhanden sein muss. Zum anderen kann die Anwesenheit eines solch großen und dicken Etiketts im Innenraum der Verpackung für den Konsumenten auch störend sein.

Eine bessere Lösung zur einfachen und wirtschaftlichen Bereitstellung eines Mittels, um zerstörungsfrei die Atmosphäre innerhalb einer Verpackung optisch ermitteln zu können, wird in der veröffentlichten US-Patentanmeldung US 2013/0177480 A1 vorgestellt. Diese Schrift schlägt eine frei positionierbares, von außen optisch messbare Druckmesssonde vor, welche eine einen Fluoreszenzfarbstoff enthaltende Feststoffkomposition auf einer Trägerschicht umfasst, wobei auf der Unterseite der Trägerschicht eine Haftschicht zum Befestigen der Druckmesssonde an der Innenseite einer Verpackung vorgesehen ist. Es wird vorgeschlagen diese vergleichsweise kleinen und aus billigen Ressourcen herstellbaren Druckmesssonden, welche auch insbesondere die optische Bestimmung der Sauerstoffkonzentration der Verpackungsatmosphäre erlauben, in großer Zahl hintereinander angeordnet, auf platzsparend aufgerollten Bändern bereitzustellen.

Der Nachteil dieser Lösung ist jedoch, dass keine Maßnahmen vorgesehen sind, eine ausreichende Genauigkeit der Fluoreszenzmessung sicher zu stellen. Insbesondere erfolgt die optische Messung der versiegelten Verpackung üblicherweise an den an stationären vorbeibewegenden Verpackungen. Um ein gleichförmiges Signal zu erhalten ist es daher wichtig, dass die Farbstoffdichte im Sichtfeld des Sensors sich während der Messzeit möglichst nicht ändert. Bei den von der US 2013/0177480 A1 vorgeschlagenen punktmesssondenförmigen Etiketten ist eine Messung sich bewegender Verpackungen kaum möglich, da die den fluoreszenzaktiven Farbstoff enthaltende Schicht zum einen eine zu kleine Fläche aufweist und zum anderen eine gleichmäßige scheinbare Farbstoffdichte nicht sichergestellt ist.

Es ist vor diesem Hintergrund die Aufgabe vorliegender Erfindung, ein Mittel zur störungsfreien Bestimmung eines Analyten, insbesondere Konzentrationsbestimmung des Analyten, in einer Verpackung breitzustellen, welches schnell und günstig herstellbar ist und auch bei einer Relativbewegung zwischen Etikett und optischem Sensor eine genaue Messung erlaubt. Weiterhin sollte die mit dem Mittel versehene Verpackung nach Möglichkeit frei und ohne Rücksicht auf die zur Ermöglichung der Konzentrationsbestimmung vorgesehenen technischen Mittel nehmen zu müssen, grafisch gestaltet und insbesondere bedruckt werden können.

Gelöst wird diese Aufgabe durch eine Verpackung mit einem Indikatoretikett gemäß Anspruch 1.

Der erste, hier nicht separat beanspruchte, Aspekt der vorliegenden Erfindung ist ein Indikatoretikett zur Verwendung in der erfindungsgemäßen Verpackung. Dieses Etikett umfasst in an sich bekannter Weise eine Trägerschicht mit einer auf der Unterseite flächig aufgebrachten Haftschicht, wobei die Trägerschicht auf der Unterseite gegenüberliegenden

Oberseite einen Indikatorsubstanzpunkt trägt. Der Indikatorsubstanzpunkt umfasst eine Indikatorsubstanz, deren Eigenschaften abhängig von der Anwesenheit und insbesondere der Konzentration des zu bestimmenden Analyten in einer Weise ändern, die von außen mit optischen Mitteln detektierbar ist. Insbesondere kann es sich bei der Indikatorsubstanz um eine lumineszenten, also fluoreszenten oder phosphoreszenten, Farbstoff handeln, der bei Anregung mit Licht einer gewissen Wellenlänge Licht einer anderen Wellenlänge ausstrahlt. Die Konzentrationsbestimmung des Analyten ist bei solchen lumineszenzbasierten Farbstoffen dadurch möglich, das die Parameter der Lumineszenz von der Anwesenheit und im Allgemeinen auch von der Konzentration des Analyten abhängig sind.

Beispielsweise verringern im Falle von Porphyrinfarbstoffen in der Gegenwart von Sauerstoff die Abklingzeit sowie die Intensität der Fluoreszenzantwort auf eine optische Anregung. Sprich in Gegenwart von Sauerstoff fällt das emittierte Fluoreszenzlicht des Farbstoffs weniger intensiv aus und das Fluoreszenzsignal klingt nach Abschalten des Anregungslichts auch schneller ab als ohne Sauerstoff. Diese Änderungen sind umso größer, je höher die Konzentration des Sauerstoffs ist. Aus der Messung von Intensität und/oder Abklingzeit des Fluoreszenzantwortsignals lässt sich somit auf die Konzentration des Sauerstoffs im Bereich des Farbstoffs schließen.

Kennzeichnend für das Indikatoretikett ist die gleichförmige Dicke des Indikatorsubtanzpunktes, welcher in manchen Ausführungsformen auch als die gesamte Oberfläche des Etiketts bedeckende Indikator(substanz)schicht, oder als länglicher Indikator(substanz)streifen ausgebildet ist, über seine Fläche. Die Abweichung der relativen Dicke des Indikatorsubstanzpunktes variiert zwischen zwei Punkten zumindest in einer zur Oberseite der Trägerschicht parallelen Längsrichtung bevorzugt um nicht mehr als 10 Prozent, besonders bevorzugt nicht mehr als 1 Prozent. Hierdurch wird sichergestellt, dass bei einer optischen Messung des Indikatoretiketts die für den optischen Sensor sichtbare Flächendichte an Indikatorsubstanz sich bei Bewegung des Etiketts durch den Erfassungsbereich des Sensors konstant bleibt. Somit ist erreicht, dass ein gleichmäßiges optisches Signal erfasst werden kann und eine die Genauigkeit der Bestimmung des
Analyten nicht durch eine Relativbewegung von Sensor und Etikett negativ beeinflusst wird.

Die Dicke der Indikatorschicht muss hierfür nicht unbedingt in zwei zueinander senkrechte Richtungen gleichförmig sein. Es ist vielmehr bereits ausreichend, wenn der Indikatorsubstanzpunkt in eine Richtung eine gleichförmige Dicke aufweist, also der Indikatorsubstanzpunkt streifenweise Bereiche gleichförmiger Dicke aufweist. Eine Dickenvariation senkrecht hierzu kann toleriert werden, wenn sichergestellt wird, dass bei der Messung eine Relativbewegung zwischen Sensor und Indikatoretikett nur Parallel zu der Richtung gleichmäßiger Dicke stattfindet.

In bevorzugten Ausführungsformen weisen die Indikatoretiketten vorteilhaft eine vergleichsweise kleine Abmessung von wenigen Millimetern auf und werden in besonders bevorzugten Ausführungsformen in großer Zahl hintereinander auf zu Rollen aufgewickelten Bändern bereitgestellt.

Die Indikatoretiketten werden nach einem Herstellungsverfahren durch Aufdrucken des Indikatorsubstanzpunktes auf einen Etikettenrohling oder Blanko-Etikett hergestellt. Hierzu wird zunächst ein Etikettenrohling bereitgestellt, welcher eine Trägerschicht und eine unterseitige Haftschicht umfasst. Praktischerweise sollte die der Trägerschicht gegenüberliegende Seite der Haftschicht in bekannter Weise mit einer an der Haftschicht nur leicht anhaftenden Abziehschicht bedeckt sein, um ein ungewolltes Verkleben des Etiketts zu vermeiden. Der Etikettenrohling kann bereits in seinem Umriss dem späteren Etikett entsprechen. Bevorzugt wird aber ein Rohling bereitgestellt, der einen Etikettenabschnitt und einen davon getrennten, an den Etikettenabschnitt angrenzenden oder diesen umgebenden Überschussabschnitt umfasst. Auf diesen Rohling wird der Indikatorsubstanzpunkt derart aufgedruckt, dass er mit mindestens einem, bevorzugt zwei insbesondere gegenüberliegenden Randbereichen über den Etikettenabschnitt hinausragt. Eine mehr oder minder gleichmäßige Verteilung der Indikatorsubstanz über die Fläche des Indikatorsubstanzpunktes wird durch die Selbstnivelierung der verwendeten Tinte erreicht. Der hinausragende, auf dem Überschussabschnitt liegende Teil wird nach dem Trocknen der aufgedruckten Indikatorsubstanz zusammen mit dem Überschussabschnitt entfernt. Zurück bleibt ein erfindungsgemäßes Etikett mit einem Indikatorsubstanzpunkt der zumindest in einer zur Oberfläche der Trägerschicht parallelen Richtung eine gleichförmige Dicke aufweist.

Als Beispiel kann ein Etikettenrohling verwendet werden, bei dem der Etikettenabschnitt eine runde oder ovale Form hat, und der auf allen Seiten von einem Überschussabschnitt umgeben ist. Ein solcher Rohling kann beispielsweise aus einem Rohling umfassend eine Trägerschicht mit unterseitiger Haftschicht und bevorzugt einer darunter befindlichen Abziehschicht hergestellt werden, indem ein Etikettenabschnitt in gewünschter Form herausgeschnitten oder gestanzt wird, wobei hierbei darauf zu achten ist, dass die Abziehschicht nicht verletzt wird, damit Etikettenabschnitt und Überschussabschnitt weiterhin in räumlicher Nähe gehalten werden. Auf einen derartigen Rohling können nun verschiedene Indikatorsubstanzen aufgedruckt werden.

In manchen Ausführungsformen wird ein den Etikettenabschnitt vollständig bedeckender und auf allen Seiten über diesen hinaus auf den Überschussabschnitt ragender Indikatorsubstanzpunkt aufgedruckt. Während des Trockenvorgangs sammeln sich die in der flüssigen Indikatorsubstanz dispergierten Feststoffteilchen tendenziell am Rande des aufgedruckten Punktes, wodurch dieser nach dem Trocknen dort seine größte Dicke hat. In der Mitte hingegen findet sich nach dem Trocknen die geringste Dicke an Indikatorsubstanz. Der Dickengradient ist jedoch weiter entfernt vom Rand, beispielsweise zehn oder mehr Prozent der Erstreckung des Indikatorsubstanzpunktes in diese Richtung, vergleichsweise gering, sodass ein ausreichender Überstand des aufgedruckten Punktes über die Ränder des Etikettenabschnittes hinaus sicherstellt, dass nach dem Entfernen, insbesondere Abziehen, des den Etikettenabschnitt umgebenden Überschussabschnittes der auf dem Etikettenabschnitt verbleibende Mittelteil des Indikatorsubstanzpunkes, welcher diesen in diesem Beispiel vollflächig bedeckt, eine weitgehend gleichförmige Dicke hat. Die gleichmäßige Dicke liegt bei diesen Ausführungsformen auch in beider zueinander senkrechten Richtung der Oberfläche vor.

In alternativen Ausführungsformen erfolgt das Auf- bzw. Unterteilen der Trägerschicht in Etikettenabschnitt und Überschussabschnitt/e auch erst nach dem Aufdrucken. Dies hat den Vorteil, dass eine sauberere Trennung zwischen dem Teil des Indikatorsubstanzpunktes auf dem Etikettenabschnitt und den (verdickten) Teilen des Indikatorsubstanzpunktes auf dem/n Überschussabschnitt/en erfolgt.

Der Nachteil bei den vorbeschriebenen Ausführungsformen der Etiketten liegt darin, dass zum Erfassen eines Etiketts, welches sich mit vergleichsweise hoher Geschwindigkeit an einem Sensor vorbeibewegt, eine gewisse räumliche Ausdehnung des Etiketts von Nöten ist. Bewegt sich das Etikett beispielsweise mit einer Geschwindigkeit von einem Meter pro Sekunde am Sensor vorbei so muss das Etikett, sofern der Sensor beispielsweise jede Millisekunde einen Datenpunkt aufnimmt und für eine zuverlässige optische Messung zehn Datenpunkte nötig sind, eine Erstreckung in Bewegungsrichtung von mindestens zehn Millimetern aufweisen. Bei einem gleichförmig mit Indikatorsubstanz bedeckten Indikatoretikett gemäß der vorbeschriebenen Ausführungsformen, welche eine runde Form hat, wäre daher eine Fläche von knapp 80 mm² mit Indikatorsubstanz zu bedecken. Die für den Anwendungsfall der Sauerstoffbestimmung verwendeten Fluoreszenzfarbstoffe sind allerdings vergleichsweise teuer, mit Preisen von einigen Tausend Euro pro Liter, sodass ein möglichst sparsamer Umgang mit ihnen aus wirtschaftlichen Gründen stark bevorzugt ist. Zudem ist eine Erstreckung quer zur Bewegungsrichtung im Umfang von zehn Millimetern weder nötig noch gewünscht. Grund ist das eine sich ändernde Breite des Indikatorsubstanzpunktes ebenfalls zu einem variierenden Messsignal führen würde. Dies könnte zwar durch Etiketten mit rechteckigem Grundriss vermieden oder bei bekannter Form der Indikatorsubstanzpunkte herausgerechnet werden. Den im zweiten Fall entstehenden Aufwand kann man sich jedoch sparen, sofern der Indikatorsubstanzpunkt eine zumindest im Wesentlichen rechteckige Form hat, wobei die längere Seite in Bewegungsrichtung weisen sollte.

Die Form des Etikettenabschnitts selbst ist im Rahmen vorliegender Erfindung von untergeordneter Bedeutung. Der Etikettenabschnitt, bzw. das fertige Indikatoretikett können rund, oval oder rechteckig sein oder eine andere sinnvolle Form aufweisen. Aus Gründen der einfacheren Herstellung und Handhabung ist eine runde oder rechteckige, beispielsweise quadratische Form naheliegend.

Auf einem Etikettenabschnitt bzw. Etikett wird der Indikatorsubstanzpunkt bevorzugt in Form eines Rechtecks aufgedruckt, wobei die beiden gegenüberliegenden Enden des Rechtecks über den Etikettenabschnitts bzw. Etiketts hinausragen und bei Herstellung gemäß des erfindungsgemäßen Verfahrens auf dem Überschussabschnitt zu liegen kommen. Nach dem Trocknen der flüssigen Indikatorsubstanz bzw. die Indikatorsubstanz(en) enthaltenden Tinte, wird der Überschussabschnitt an beiden Seiten entfernt, sodass nur ein, mit Ausnahme von gegebenenfalls gekrümmten kurzen Stirnseiten, rechteckiger und nahezu quaderförmiger Streifen auf der Oberseite der Trägerschicht verbleibt.

In Längsrichtung weißt dieser Indikatorsubstanzstreifen die erfindungsgemäß gewünschte gleichförmige Dicke auf. Quer hierzu variiert die Dicke zwar, dies ist jedoch unerheblich, wenn das Etikett beim Einsatz in der Weise in die Verpackung eingeklebt wird, dass die Längsseite des rechteckigen Indikatorsubstanzstreifens parallel zur Bewegungsrichtung der Verpackung in der Verpackungsmaschine ausgerichtet wird.

Nach dem hier offenbarten Verfahren können einzelne der für die erfindungsgemäße Verpackung vorgesehenen Indikatoretiketten hergestellt werden. Bevorzugt werden die Etiketten jedoch am Band gefertigt, in dem entsprechend dem ebenfalls beanspruchten Herstellungsverfahren für ein Indikatoretikettenband, ein Rohling in Form eines Bandes aus einer Haftschicht, welche zwischen einer oberseitigen Trägerschicht und einer unterseitigen Abziehschicht eingebettet ist, bereitgestellt wird, und in diesem Rohling gleichzeitig oder nacheinander hintereinanderliegende Etikettenabschnitte in gewünschter Form abgetrennt werden. Dieses Abtrennen kann durch Stanzen oder durch Schneiden, beispielsweise Laserschneiden erfolgen, wobei bei diesem Prozess darauf zu achten ist, nur die Träger- und gegebenenfalls auch die Haftschicht zu durchtrennen, die Abziehschicht jedoch unbeschädigt zu lassen. Um dies herzustellen ist es vorteilhaft, für die Haft-/Abziehschicht ein Material zu wählen, welches das verwendete Abtrennverfahren nicht so leicht durchtrennbar ist wie die Trägerschicht. Der, sofern er nicht weiter unterteil wird, zusammenhängende

Trägerschichtbereich welcher die hintereinander liegenden Etikettenabschnitte umgibt bildet dann den Überschussabschnitt.

In vorbeschriebener Weise werden bei dem hier offenbarten Verfahren Indikatorsubstanzpunkte auf die Etikettenabschnitte derart aufgedruckt, dass sie an einer Stelle, bevorzugt an zwei Stellen, insbesondere zwei gegenüberliegenden Stellen, oder an manchen Ausführungsformen kommen an allen Seiten, über den Etikettenabschnitt hinausragen, sodass sich die Ränder insoweit auf dem Überschussabschnitt befinden. Die Indikatorsubstanztinte wird nach dem Aufdrucken trocknen gelassen oder aktiv getrocknet und nach dem Trocknen der Überschussabschnitt entfernt. Um dies bei der industriellen Fertigung zu vereinfachen kann statt eines großen, zusammenhängenden Überschussabschnittes dieser, beispielsweise im Zuge des Abtrennvorgangs der Etikettenabschnitte, in kleinere leichter maschinell entfernbare Teile unterteilt werden. Übrig bleibt nach dem Entfernen des Überschussabschnittes ein Band mit darauf angeordneten erfindungsgemäßen Indikatoretiketten aus Trägerschicht, unterseitiger flächig aufgebrachter Haftschicht und einem Indikatorsubstanzpunkt auf der Oberseite der Trägerschicht. Der Indikatorsubstanzpunkt kann hierbei wie oben beschrieben die ganze Oberseite der Trägerschicht bedecken, oder nur einen Teil davon, beispielsweise in Form eines Rechteckes rechts und links Bereiche des Etikettenabschnitts unbedeckt lassen. An eine, bevorzugt an zwei gegenüberliegenden Seiten schließt der Indikatorsubstanzpunkt jedoch bevorzugt bündig mit dem Etikett bzw. den Etikettenabschnitt ab.

Das Aufdrucken der Indikatorsubstanzpunkte kann vor oder nach dem Abtrennen der Etikettenabschnitte erfolgen.

Gegenstand der hier beanspruchten Erfindung ist eine Verpackung für ein Lebensmittel oder ein anderes empfindliches Gut, beispielsweise eine Verpackung mit einer modifizierten Atmosphäre, in welcher an einer von außen einsehbaren Stelle ein vorbeschriebenes Indikatoretikett eingeklebt ist. Bei der Verpackung handelt es sich um eine mit einem oberseitigen Film verschlossenen Schalenverpackung. Der Begriff "von außen einsehbar" ist hier so zu verstehen, dass die Stelle für von außen beaufschlagtes Anregungslicht und gleichzeitig auch für von innen auftreffendes, von der Indikatosubstanz in dem Indikatorsubstanzpunkt des Indikatoretiketts ausgesendeten Fluoreszenzlicht, welches üblicherweise eine andere (niedrigere) Frequenz als das Anregungslicht aufweist, hinreichend transparent ist.

Die erfindungsgemäße Verpackung kann an der einsehbaren, also hinreichend transparenten, Stelle unbedruckt sein oder einen Aufdruck aufweisen, der jedoch so gestaltet ist, dass er für Anregungs- und Fluoreszenzlicht ausreichend transparent ist. Dies kann dadurch erreicht werden, dass für den Aufdruck eine für das Anregungs- und Fluoreszenzlicht transparente Tinte verwendet wird.

Alternativ oder zusätzlich kann der Aufdruck durchgehend, zumindest aber in dem Bereich der Fläche, die (auf der Innenseite der Verpackung) von dem Indikatoretikett beansprucht wird, bedruckte und nicht bedruckte Bereiche aufweist, wobei die nichtbedruckten Bereich mindestens 5%, bevorzugt mindestens 10%, insbesondere mindestens 25% der Fläche des Indikatoretiketts ausmachen. Bedruckte und nicht-bedruckten Bereiche wechseln sich bevorzugt ab, insbesondere in Form eines eindimensionalen (Strich-) oder zweidimensionalen (Punkt-)Rasters. Die nicht-bedruckten Bereiche haben dabei alle eine Höhe und Breite, welche größer als die Wellenlänge des Anregungs- und auch des (längerwelligen) Fluoreszenzlichts ist.

In besonderen Ausführungsformen der Verpackung wird der Aufdruck mittels Digitaldruck aufgebracht und umfasst ein Raster von Rasterpunkten ist mit einem Abstand zwischen benachbarten Punkten des Rasters von mindestens dem 1,05 fachen des Radius eines Rasterpunktes, insbesondere von mindestens dem 1,1 - fachen, beispielsweise dem 1,2 - 2,0 fachen eines Rasterpunktradius.

Ein dritter, hier nicht beanspruchter, Aspekt vorliegender Erfindung ist eine Verpackungsmaschine zum Verpacken von Lebensmitteln oder anderen empfindlichen Gütern in einer Verpackung gemäß des vorherigen Aspektes, wobei die Maschine Etikettiermittel aufweist, vor dem Versiegeln der Verpackung ein erfindungsgemäß Indikatoretikett auf die mit der Atmosphäre im Inneren der Verpackung in

Kontakt kommenden Innenseite an einer geeigneten, insbesondere transparenten, Stelle eines erfindungsgemäßen Indikatoretikett aufzukleben.

Darüber hinaus umfasst die hier offenbarte Verpackungsmaschine bevorzugt mindestens einen optischen Sensor zum Erkennen und Ausmessen von Indikatoretiketten in den versiegelten Verpackungen. Mittels dieses Sensors kann nach der Herstellung, abhängig vom verwendeten Indikatoretikett sowie Sensor, mindestens einen Analyten, etwa eine Gaskomponente einer modifizierten Atmosphäre, im Inneren der Verpackung und so die Güte der Atmosphäre bestimmt werden. Hieraus lassen sich weitere Informationen ableiten, etwa eine zu erwartende Mindesthaltbarkeit. Dieses lässt sich im Prinzip auch auf flüssigkeitsgefüllte Verpackungen ausdehnen, sofern geeignete Indikatorsubstanzen und -etiketten eingesetzt werden.

In manchen Ausführungsformen sind mehrere optische Sensoren vorhanden. Diese können transversal zur Transportrichtung der Verpackungen durch die Maschine im Wesentlichen nebeneinander angeordnet sein um mehrere Bahnen von Verpackungen gleichzeitige messen zu können, ohne dass der Sensor seine Position schnell verändern müsste. Alternativ oder zusätzlich sind auch in Transportrichtung für jede Verpackungsbahn mindestens zwei optische Sensoren vorhanden, beispielsweise um die Atmosphäre jeder Verpackung zu zwei Zeitpunkten nach der Versiegelung Messen zu können. Um Undichtigkeiten aufzuspüren, kann zwischen den optischen Sensoren eine Rüttel- oder eine Druckwechselstrecke liegen.

Da in der Praxis verwendete Verpackungen häufig nicht voll transparent sind, sondern transparente Abschnitte sich mit bedruckten oder anderweitig abgedeckten Abschnitten abwechseln, und die transparenten, für das Aufkleben des erfindungsgemäßen Etiketts geeigneten Abschnitte im allgemeinen für jede Verpackung an einer anderen Stelle liegen, ist es wichtig, dass die Etikettiermittel und auch der optische Sensor in transversaler Richtung auf die transversale Position der transparenten Abschnitte in denen die Indikatoretiketten aufgeklebt werden sollen, ausrichtbar sind. Diese Ausrichtung kann durch händisches Verstellen der Etikettiermittel und/oder des optischen Sensors, vor einem Durchlauf mit einer bestimmten Verpackungsgestaltung vorgenommen werden. Alternativ oder zusätzlich sind die Etikettiermittel und/oder der optische Sensor in transversaler Richtung fahrbar montiert, etwa an einem auf einer transversalen Schiene laufenden Schlitten.

Auch bei einer gegebenen Ausgestaltung der Verpackung sind Toleranzen auszugleichen, innerhalb derer die transparenten Bereiche transversal wie longitudinal von der erwarteten Position abweichen. Daher sind in vorteilhaft weitergebildeten Ausführungsformen der Maschine optische Erfassungsmittel vorgesehen, welche die genaue Positionen der transparenten Bereichs individuell für jede Verpackung bestimmen. Die Etikettiermittel und/oder der optische Sensor richten sich dann für jede der Verpackungen in transversaler Richtung anhand der verpackungsindividuell bestimmten Position aus, so dass für jede Verpackung das Etikett durch das jeweilige Etikettiermittel an exakt der gleichen Stelle relativ zum Rand des jeweilig anvisierten transparenten Bereichs geklebt wird und der, vergleichsweise enge, Erfassungsbereich des optischen Sensors an der lateralen Position des Etiketts ist. Hierzu ist es nötig, dass, wie für die oben beschriebenen Ausführungsformen als Option vorgestellt, die Etikettiermittel bzw. ggf. auch der optische Sensor transversal verfahrbar montiert sind..

Transparent bezieht sich hier und auch sonst in dieser Anmeldung nicht notwendigerweise auf eine Durchsichtigkeit im sichtbaren Spektrum sondern, sofern nicht ausdrücklich anders erwähnt, auf Durchsichtigkeit der Verpackung in dem/den für die optische Messung der Indikatorsubstanz maßgeblichen Wellenlängenbereich/en.

Weiterhin bevorzugt setzt die hier offenbarte Verpackungsmaschine Indikatoretikettenbänder mit hintereinander angeordneten Indikatoretiketten ein. Diese können beispielsweise auf einem Förder(band)arm abgerollt werden, welche an einem distalen Ende ein Mittel zum Ablösen der Etiketten und Aufbringen an einer gewünschten Stelle einer Verpackung aufweist. Die Mittel zum Ablösen können etwa eine Umlenkrolle mit geringem Radius umfassen, sodass, wenn das Indikatoretikettenband mit dem Etikett auf der gegenüberliegenden Seite der Abziehschicht über die Umlenkrolle läuft, das Etikett aufgrund des geringen Radius der Umlenkrolle und abhängig von der Steifigkeit der Trägerschicht sich an einer Seite ablöst, sodass es mit der abgelösten Seite an der erkannten und bevorzugten Stelle mit der Verpackung in Kontakt gebracht und durch weiteres Ablösen vom Etikettenband schließlich vollständig auf die Verpackung transferiert werden kann.

Die Spitze des Förderbandarmes kann in diesen bevorzugten Ausführungsformen zumindest in eine Richtung quer zur Verfahrrichtung der Verpackungsmaschine positioniert werden, um einen auf einer Verpackung anzubringendes Etikett in den Bereich zu fahren, in dem das optische Erfassungsmittel den hierfür geeigneten bzw. anvisierten transparenten Bereich erkannt hat. Dies ist etwa dadurch möglich, dass der arm um seine z- oder Gierachse schwenkbar gelagert ist. Alternativ oder zusätzlich kann der Arm auch in eine transversale Richtung verfahrbar gelagert sein, etwa mittels eines Schlittens. Es ist darüber hinaus denkbar, dass das distale Ende des Förderbandarmes in beiden Richtungen innerhalb der Verpackungsebene repositionierbar ist, also zusätzlich auch in die vertikale Richtung. Hierfür könnte der Arm etwa um seine y- oder Nickachse drehbar gelagert oder in vertikaler Richtung verfahrbar sein.

Das hier offenbarte Verfahren zum Verpacken von Lebensmitteln oder anderer empfindlicher Güter gemäß dieses Aspekts vorliegender Erfindung in Verpackungen gemäß des zweiten Aspekts der Erfindung umfasst die beiden Schritte, dass zunächst mittels der Etikettiermittel der Verpackungsmaschine ein Indikatoretikett gemäß des ersten Aspekts der Erfindung an der später mit der Atmosphäre im Inneren der versiegelten Verpackung in Kontakt stehenden Seite eines beliebig vorgebbaren oder vorgegebenen transparenten Bereichs angebracht wird. Beliebig vorgegeben bedeutet hier, dass die Etikettiermittel relativ zu einem Bezugspunkt der Verpackungen frei ausgerichtet werden können und zwar zumindest vor jedem Durchlauf mit einer bestimmten Verpackungsgestaltung oder noch besser, dynamisch während eines Durchlaufs, etwa um verschiedene Verpackungsgestaltungen innerhalb desselben Durchlaufs mit einer Verpackungsmaschine bearbeiten zu können oder um Fertigungsbedingte Toleranzen in der Position der für die Indikatoretiketten vorgesehenen transparenten Bereiche der Verpackung auszugleichen.

In einem zweiten Schritt wird mittels des manuell oder selbsttätig auf den beliebig vorgegebenen Bereich der Verpackungen ausgerichteten optischen Sensors eine Messung des Analyten, etwa einer Gaskomponente, innerhalb der Verpackung durchgeführt. Hierdurch kann eine Nachkontrolle der Atmosphäre und eine Bestimmung der zu erwartenden Haltbarkeit erreicht werden. Zusätzlich kann auch die Versiegelungsqualität kontrolliert werden, wenn für jede Verpackung in einem anderen zeitlichen Abstand eine zweite Messung des oder der Analyten, insbesondere Gaskomponente/n stattfindet, entweder durch räumliches Verfahren des optischen Sensors oder einfach durch Einsatz eines zweiten, in Transportrichtung weiter hinten angeordneten optischen Sensors.

Weitere vorteilhafte Weiterbildungen sind in den abhängigen Ansprüchen enthalten und sollen im Folgenden im Einzelnem wiedergegeben. Sie sind auch in beliebiger Kombination miteinander Teil der vorliegenden Erfindung, sofern sie sich nicht ersichtlich gegenseitig ausschließen.

Bei dem Indikatoretikett gemäß des ersten Aspekts vorliegender Erfindung liegt die relative Dickenabweichung zumindest in der einen, bevorzugt in beiden zu der Oberseite der Trägerschicht senkrechten Richtungen bei nicht mehr als 10%, insbesondere nicht mehr als 1%. Die bevorzugte absolute Dicke der Indikatorschicht bzw. Indikatorschichtpunktes ist zwischen 5 und 500 Mikrometer, insbesondere zwischen 1 und hundert Mikrometern.

Die Trägerschicht des Indikatoretiketts kann aus Zellulose oder Kunststoff, insbesondere Polypropylen oder Polyester bestehen und hat bevorzugt eine Dicke zwischen 20 und 1000 Mikrometer insbesondere bevorzugt zwischen 50 und 200 Mikrometer.

Die Haftung des ordnungsgemäßen Indikatoretiketts an der Innenseite der Verpackung vermittelnden Haftschicht enthält als Haftmittel bevorzugt einen Lebensmittel verträglichen Klebstoff, bzw. besteht aus einem solchen.

Der Indikatorsubstanzpunkt auf der Oberseite der Trägerschicht eines Indikatoretiketts ist bevorzugt länglich, mit einem Länge-zu-Breite Verhältnis von zwischen 2 und 20, bevorzugt zwischen 5 und 10. Insbesondere bevorzugt ist der Indikatorsubstanzpunkt von rechteckiger Form mit einer Breite von zwischen 0,5 und 2 Millimetern und einer Länge von zwischen 4 und 10 Millimetern. In besonders bevorzugten Ausführungsformen ist der Indikatorsubstanzpunkt zirka einen Millimeter breit und zirka 7 oder 8 Millimeter lang.

Der Indikatorsubstanzpunkt schließt in manchen Ausführungsformen des Indikatoretiketts an mindestens einer Stelle, bevorzugt an zwei, insbesondere gegenüberliegenden Stellen bündig mit dem Rand der Trägerschicht ab. Hierdurch wird die Länge des Indikatorsubstanzpunktes oder -streifens maximiert.

Die Haftschicht hat bevorzugt eine Dicke von zwischen 10 und 200 Mikrometern, insbesondere zwischen 50 und 100 Mikrometern.

Das Indikatoretikett ist in bevorzugten Ausführungsformen für sichtbares Licht im Wesentlichen transparent und insbesondere bevorzugt auch farblos, sodass es bei Einbringen im Inneren einer Verpackung für den die Verpackung öffnenden Verbraucher möglichst wenig auffällig ist und somit das Gesamtbild der Verpackung nicht oder kaum stört.

In bevorzugten Ausführungsformen ist die Indikatorsubstanz in dem Indikatorsubstanzpunkt ein lumineszenter, insbesondere fluoreszenter, Farbstoff bei dem mindestens ein Lumineszenzparameter wie etwa relative Intensität bei Anregung, Anregungsfrequenz, Emissionsfrequenz und/oder Abklingzeit von der Anwesenheit des Analyten abhängig ist. Insbesondere ändern sich mindestens eine oder auch mehrere dieser Parameter kontinuierlich mit der Konzentration des Analyten in dem zu analysierenden Fluid, welches insbesondere ein Gasgemisch sein kann.

Die erfindungsgemäße Verpackung mit einem Indikatoretikett besteht aus einem flächigen oder schalenförmigen Unterteil, welches oberseitig durch eine zumindest teilweise transparente Folie verschlossen ist. Das erfindungsgemäße Indikatoretikett ist bevorzugt an der Unterseite der oberseitigen Folie angeklebt.

In manchen Ausführungsformen der hier offenbarten Verpackungsmaschine ist der mindestens eine optische Sensor dazu vorbereitet, manuell in transversaler Richtung relativ zu einem seitlichen Rand der versiegelten Verpackungen ausgerichtet zu werden. Bevorzugt kann er dies auch selbsttätig und ist hierzu insbesondere transversal verfahrbar montiert.

Weiterhin bevorzugt scannt der Sensor nach dem Ausrichtung nach durch seinen Sicht-/Erfassungsbereich fahrenden Indikatoretiketten. Hierzu können etwa in dem der Erfassungsbereich in einer niedrigeren Scanrate abgetastet wird. Sofern beim Scannen ein solches Indikatoretikett erkannt wird, wird eine optische Messung des Etiketts vorgenommen, etwa in dem eine Abtastung des Erfassungsbereichs mit einer im Vergleich zur Scanrate erhöhten Messrate erfolgt. Die Scanrate kann etwa zwischen 50 und 500 Hz, bevorzugt 100 bis 200 Hz und die Messrate kann zwischen 100 und 2000 Hz, bevorzugt zwischen 500 und 1000 Hz betragen.

In weiterhin bevorzugten Ausführungsformen gibt der Sensor eine Warnung oder einen Hinweis aus, wenn beim Scannen ein erwartetes Indikatoretikett nicht erfasst werden kann, etwa weil es ganz fehlt oder durch die Etikettiermittel außerhalb oder nur teilweise innerhalb des transparenten Bereichs eingeklebt wurde. Wann der Sensor ein Indikatoretikett erwartet kann anhand der Zeitfenster bestimmt werden, in welchem die transparenten Bereiche der einzelnen Verpackungen den Messbereich durchqueren. Dies kann anhand der bekannten Erstreckung der transparenten Bereiche in longitudinaler Richtung sowie der Transportgeschwindigkeit der Verpackung und eines definierten Anfangspunktes vorherberechnet werden. Alternativ oder zusätzlich werden die Zeitfenster anhand der mittels optional zusätzlich vorhandener optischer Erkennungsmittel bestimmten Positionen und dem bekannten Abstand der Erkennungsmittel entlang der Transportrichtung der Verpackungen bzw. der von den Erkennungsmitteln überwachten Verpackungsteilen, etwa Formaten von Verpackungsunterteilen oder die zugehörigen Versiegelungsfolien, errechnet.

Weitere Merkmale, Eigenschaften und Vorteile dieser Erfindung ergeben sich aus den nachfolgend unter Bezugnahme auf die Figuren vorgestellten Ausführungsbeispielen. Diese sollen vorliegende Erfindung lediglich illustrieren und in keiner Weise in ihrer Allgemeinheit einschränken.

Im Einzelnen zeigen:
- FIG. 1A:: Einen schematischen Schnitt durch ein für eine erfindungsgemäße Verpackung vorgesehenes Indikatoretikett gemäß einer ersten Ausführungsform.
- FIG. 1B - C:: Draufsichten auf verschiedene Varianten der ersten Ausführungsform des Indikatoretiketts.
- FIG. 2A:: Einen schematischen Schnitt durch ein Indikatoretikett gemäß einer zweiten Ausführungsform.
- FIG. 2B - C:: Draufsichten auf verschiedene Varianten der zweiten Ausführungsform des Indikatoretiketts.
- FIG. 3A:: Schnitt durch einen Indikatoretikettenrohling der den Ausgangspunkt eines vorliegend nicht beanspruchten Verfahrens zur Herstellung eines Indikatoretiketts bildet.
- FIG. 3B:: Eine Draufsicht auf den Indikatoretikettenrohling aus FIG. 3A, bei der die Trennlinie zwischen Etikettenbereich und Überschussbereich sowie der Umriss des aufzudruckenden Indikatorpunktes angedeutet sind.
- FIG. 3C:: Eine Draufsicht auf den Indikatoretikettenrohling aus FIG. 3B nachdem Abtrennen des Etikettenbereiches und Aufdrucken des Indikatorsubstanzpunktes.
- FIG. 3D:: Einen Längsschnitt durch den Indikatoretikettenrohling aus FIG. 3C entlang der Linie D-D.
- FIG. 3E:: Perspektivische Ansicht eines fertigen Indikatoretiketts unmittelbar nach dem Entfernen des Überschussbereichs.
- FIG. 4:: Schematisch ein erfindungsgemäßes Indikatoretikettenband im aufgerollten Zustand mit einer Vielzahl an hintereinander angeordneten Indikatoretiketten auf einer bandförmigen Abziehschicht.
- FIG. 5A:: In schematischer Perspektive den Anfangsabschnitt eines Indikatoretikettenbandrohlings als Ausgangspunkt der Herstellung eines Indikatoretikettenbandes.
- FIG. 5B:: Eine schematischer Draufsicht auf den Anfangsabschnitt des Indikatoretikettenbandrohlings aus FIG. 5A nach dem Unterteilen der Trägerschicht in einen Überschussabschnitt und vier runde Etikettenabschnitte sowie Aufdrucken von jeweils einem Indikatorsubstanzpunkt auf jeden der Etikettenabschnitte.
- FIG. 5C:: Einen schematischen Längsschnitt durch den Indikatoretikettenbandrohling entlang der Linie C-C in FIG. 5B.
- FIG. 5D:: Einen schematischen Querschnitt entlang der Linie D-D in FIG. 5B.
- FIG. 5E:: In schematischer Perspektive den Anfangsabschnitt des fertiggestellten Indikatoretikettenbandes nach dem Entfernen des Überschussabschnittes des Indikatoretikettenbandrohlings aus FIG. 5B.
- FIG. 6:: Einen schematischen Schnitt durch eine erfindungsgemäße Verpackung mit einem erfindungsgemäßen Indikatoretikett im Inneren.
- FIG. 7:: In perspektivische Darstellung eine schematische Wiedergabe einer Ausführungsform einer Verpackungsmaschine zur Herstellung von Verpackungen wie etwa der in FIG. 6 gezeigten unter Einsatz von Indikatoretikettenanbringmitteln.

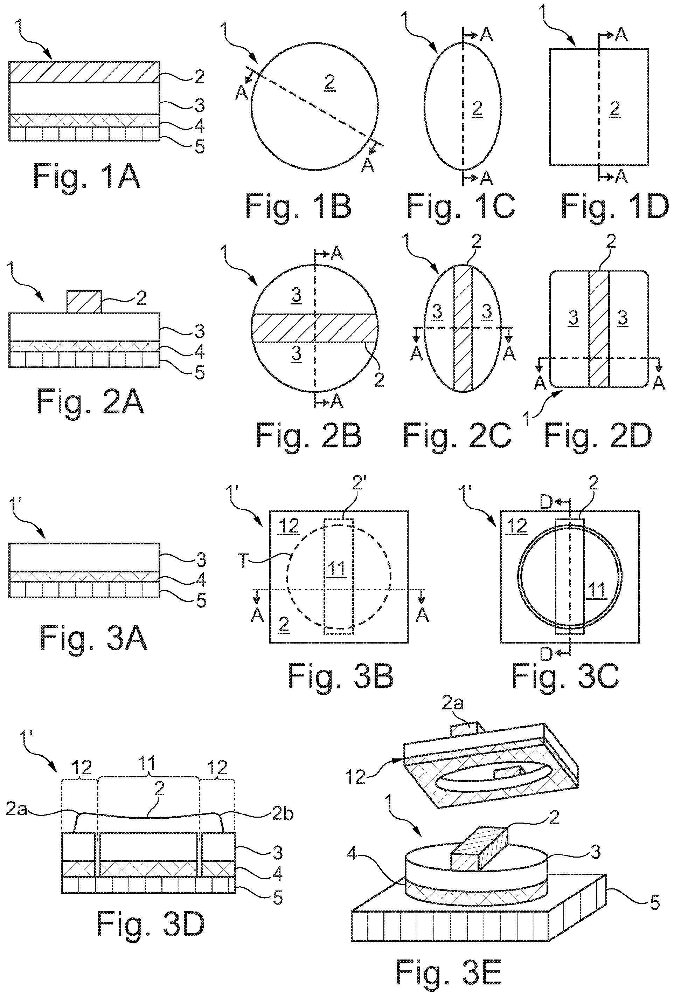

Die **Figuren 1A - 1D** zeigen verschiedene schematische Ansichten mehrerer Varianten einer erste Ausführungsform des für die erfindungsgemäße Verpackung vorgesehenen Indikatoretiketts. In den **Figuren 1B - 1D** sind drei mögliche Varianten das Indikatoretikett in Draufsicht gezeigt. Das Indikatoretikett 1 hat jeweils in **Figur 1B** einen kreisrunden, in **Figur 1C** einen ovalen oder elliptischen und in **Figur 1D** einen rechteckigen Grundriss. Andere Formen des Grundrisses wären ebenfalls möglich, etwa ein quadratischer oder ein rechteckiger Grundriss mit oder ohne mehr oder wenig stark abgerundeten Ecken.

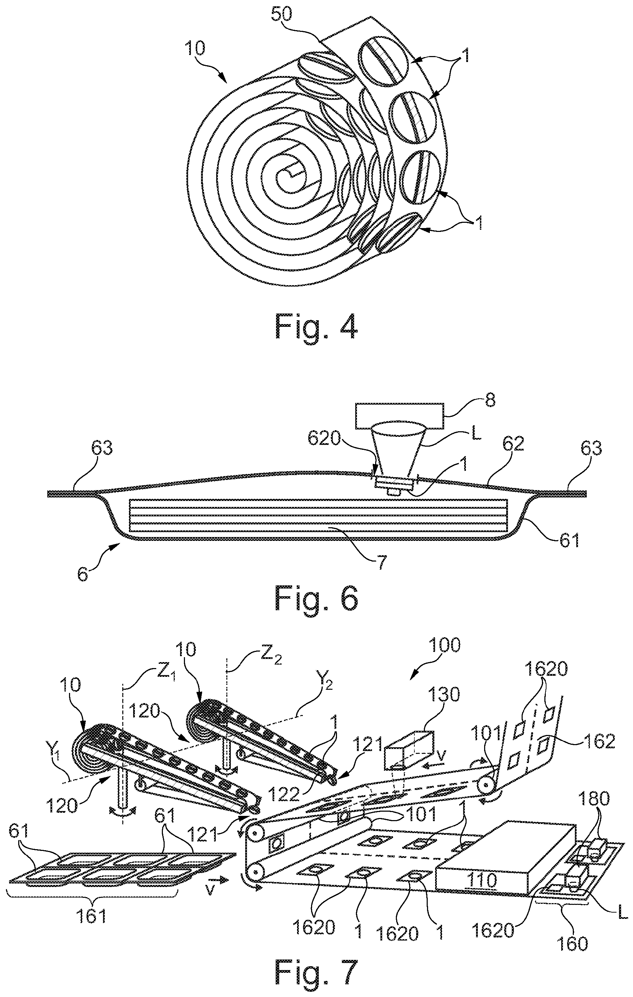

Gemeinsam ist allen Varianten der in **Figur 1A** gezeigte Querschnitt, der entlang der in den **Figuren 1B** - **1D** gezeigten gestrichelten Linien A-A genommen wurde. Das Indikatoretikett umfasst einen Schichtaufbau aus einer Trägerschicht 3, welche unmittelbar oberhalb einer Haftschicht 4 liegt. Die Haftschicht dient der Haftvermittlung beim Anbringen an die Innenseite einer zu überwachenden Verpackung (vgl. auch Figur 6 und untenstehende Beschreibung). Die Haftschicht wird vor dem Einsatz des Indikatoretiketts 1 aus rein praktischen Gründen von einer Abziehschicht 5 terminiert. Vor dem Anbringen, also Ankleben oder Anheften des Indikatoretiketts wird die Abziehschicht 5 entfernt. Die Abziehschicht 5 besteht zum leichteren Abziehen zumindest auf der der Haftschicht zugewandten Seite aus einem Material, an welchem der Haftvermittler/Klebstoff der Haftschicht 4 nur leicht anhaftet. Die Haftschicht 4 kann auch ganz aus einem solchen leicht anhaftenden Material bestehen. Beispielsweise kann die Haftschicht aus einem Fluor-terminierten Kunststoff, beispielsweise PTFE bestehen. Alternativ auch beispielsweise aus mit PTFE beschichtetem Papier oder Karton.

Die Trägerschicht 3 dient der mechanischen Abstützung des Indikatorsubstanzpunktes 2, welcher in dieser Ausführungsform die gesamte Oberseite Indikatoretiketts 1 einnimmt und daher hier auch als Indikatorsubstanzschicht oder kurz Indikatorschicht 2 bezeichnet werden kann. Die Indikatorsubstanzschicht 2 schließt somit auf allen Seiten bündig mit der darunterliegenden Trägerschicht 3 ab.

Die Indikatorschicht 2 umfasst eine für den zu messenden Analyten in dem Sinn sensitive Indikatorsubstanz, dass sich in Gegenwart des Analyten eine optisch zu erfassende Eigenschaft der Indikatorsubstanz ändert. Beispielsweise kann sich durch Anlagerung des oder Reaktion mit dem Analyten die Farbe, die Struktur, die Absorptions- oder die Emissionseigenschaften oder die Art, wie die Indikatorsubstanz polarisiertes Licht reflektiert ändern.

Die Indikatorsubstanz kann auch ein lumineszenter, also fluoreszenter oder phosphoreszenter, Farbstoff sein, der bei Anregung mit Licht einer bestimmten Frequenz als Lumineszenzantwort Licht einer anderen, üblicherweise niedrigeren, Frequenz ausstrahlt. Nach Abschalten der Anregung klingt die Lumineszenzantwort gewöhnlich exponentiell mit einer gewissen Zeitkonstante ab.

In Ausführungsformen des Indikatoretiketts welche zur Messung von Sauerstoff als Analyt bestimmt sind, umfasst der Indikatorsubstanzpunkt etwa Porphyrine als fluoreszenten Farbstoff. Bei dieser Substanzklasse Wird die Fluoreszenzantwort mit zunehmender Sauerstoffkonzentration unterdrückt, so dass zum einen die Intensität des emittierten Lichts bei gegebener Anregung abnimmt und zum anderen sich auch die Abklingzeit nach Abschalten der Anregung verkürzt. Durch Messung von Intensität und/oder Abklingzeit kann daher nach vorheriger Kalibrierung auf die Sauerstoffkonzentration in der mit dem Indikatoretikett in Kontakt stehenden Atmosphäre geschlossen werden.

Die Indikatorschicht kann auch mehrere, verschiedene Indikatorsubstanzen umfassen. Die verschiedenen Indikatorsubstanzen können für verschiedene Analyten sensitiv sein, um die Anwesenheit und/oder die Konzentration mehr als eines Analyten optisch messen zu können. Zwei oder mehr der Indikatorsubstanzen einer mehrkomponentigen Indikatorschicht können auch für den selben Analyten sensitiv sein, jedoch in verschiedener Weise und/oder verschieden stark um eine redundante Messung zu erlauben oder mit verschiedenen optischen Sensoren kompatibel zu sein.

Der Vorteil der vollständigen Bedeckung der Oberseite des Indikatoretiketts mit einem als Indikatorschicht ausgeführten Indikatorsubstanzpunktes liegt darin, dass zum einen eine größere Fläche zur optischen Erfassung vorhanden ist, als wenn nur ein Teil der Oberseite bedeckt wäre. Hierdurch kann die Konzentrationsbestimmung genauer sein. In Kombination mit dem untenstehend näher erläuterten Herstellungsverfahren für Indikatoretikette ergibt sich der zusätzliche Vorteil, dass eine Indikatorschicht gleichmäßigerer Dicke geschaffen werden kann. Genauer ist es bei der Herstellung eines Indikatoretiketts gemäß der ersten Ausführungsform der **Figuren 1A - 1D** unter Einsatz des Herstellungsverfahrens möglich, die Dickenabweichungen in zwei statt nur einer Raumrichtung gering zu halten, beispielsweise unterhalb einer relativen Dickenabweichung von 10% oder weniger oder 1% oder weniger.

In den **Figuren 2A - 2D** sind verschiedene schematische Ansichten von Varianten eines Indikatoretiketts gemäß einer zweiten bevorzugten Ausführungsform gezeigt.

Der Aufbau, inklusive des in der **Figur 2A** illustrierten Schichtaufbaus und der in den **Figuren 2B - 2D** dargestellten möglichen Grundrisse, des Indikatoretiketts dieser zweiten Ausführungsform entspricht dem der ersten Ausführungsform, mit dem Unterschied, dass auf der Oberseite der Trägerschicht 3 ein streifenförmiger Indikatorsubstanzpunkt 2 statt einer die gesamte Oberseite bedeckenden Indikatorsubstanzschicht wie bei der ersten Ausführungsform vorhanden ist. Eine weitere Abweichung sind die (stärker) abgerundeten Ecken des Grundrisses der Figur 2D.

Der Indikatorstreifen 2 der zweiten Ausführungsform schließt an den gegenüberliegenden kurzen Stirnseiten bündig mit der Trägerschicht 3 ab, lässt aber jenseits beider gegenüberliegender langen Seiten einen Teil der Trägerschicht 3 frei. Hierdurch ist die vom Indikatorsubstanzstreifen 2 bedeckte Oberfläche sowie das Volumen an Indikatorsubstanz insgesamt deutlich kleiner, als in der ersten Ausführungsform, wodurch in dieser Ausführungsform pro Indikatoretikett vorteilhaft weniger von der, üblicherweise kostspieligen Indikatorsubstanz verwendet wird. Um dennoch ein zuverlässiges Erfassen und Messen der optischen Veränderung der Indikatorsubstanz bei einer Relativbewegung zwischen Etikett 1 und optischem Sensor zu ermöglichen, wurde der Indikatorsubstanzpunkt als Indikatorsubstanzstreifen 2 ausgeführt, welcher so lange wie möglich ist und daher bündig mit dem Rand der Trägerschicht abschließt. Beim Einsatz des Etiketts ist dann nur darauf zu achten, dass dieses derart in das zu messende Objekt, etwa eine Verpackung, eingeklebt wird, dass die lange Seite des Indikatorstreifens 2 so weit wie möglich parallel zu der Relativbewegungsrichtung liegt.

In den **Figuren 3A - 3E** wird das Herstellungsverfahren am Beispiel der Herstellung eines Indikatoretiketts gemäß der zweiten Ausführungsform illustriert.

Das Verfahren beginnt mit der Bereitstellung oder anderweitigen Herstellung eines Indikatoretikettenrohlings 1', der in **Figur 3A** in einer schematischen Schnittzeichnung und in Figur 3 B in Draufsicht zu sehen ist. Der Rohling 1' umfasst die in dieser Reihenfolge übereinanderliegenden Schichten Abziehschicht 5, Haftschicht 4 und Trägerschicht 3, deren Eigenschaften, wie Zusammensetzung und Dicke, denen im Zusammenhang mit dem Indikatoretikett der ersten und zweiten Ausführungsformen entsprechen können.

Die Draufsicht der **Figur 3B** zeigt mittels der gestrichelten Linie die vorzunehmende Aufteilung in beispielhaft kreisrunden Etikettenabschnitt 11 und den diesen umgebenden, komplementär geformten Überschussabschnitt 12. Mit einer gepunkteten Linie 2' ist superponiert die nach dem Abtrennen/Unterteilen auf die Oberseite der Trägerschicht aufzudruckende streifenförmige Indikatorsubstanzpunkt 2' angedeutet. Der in Figur 3A dargestellte Schnitt wurde beispielhaft entlang der Schnittlinie A-A der Figur 3B genommen.

In der **Figur 3C** ist die Draufsicht des Rohlings 1' nach Durchführung der beiden Schritte Abteilen des Etikettenabschnitts 11 vom Überschussabschnitt 12 und Aufdrucken des Indikatorstreifens 2 gezeigt. Wie dort zu sehen, wird der Indikatorstreifen 2 derart aufgedruckt, dass sein Mittelpunkt mit dem Mittelpunkt des Etikettenabschnitts 11 zusammenfällt und seine beiden gegenüberliegenden kurzen stirnseitigen Endbereiche auf dem Überschussabschnitt 12 zu liegen kommen.

Der Vorteil dieser Ausgestaltung wird anhand der **Figur 3D** ersichtlich, welche den Rohling 1' aus der Figur 3C im Längsschnitt entlang der Linie D-D zeigt. Zu erkennen ist zum einen, dass sich die Unterteilung in Etikettenabschnitt 11 und Überschussabschnitt 12 nur durch die Trägerschicht 3 und die Haftschicht 4 zieht, die Abziehschicht 5 jedoch nicht unterteilt ist. Dies hat den Vorteil, dass die auch nach der Unterteilung Etikettenabschnitt und Überschussabschnitt für das Aufdrucken des Indikatorstreifens 2 in der Nähe zueinander gehalten werden.

In alternativen Ausführungsformen des Verfahrens, erfolgt das Aufdrucken des Indikatorstreifens vor der Unterteilung des Rohlings 1' in die beiden Abschnitte Etikettenabschnitt 11 und Überschussabschnitt 12. In diesen Ausführungsformen ist es auch möglich, die Abziehschicht 5 mit zu durchtrennen und somit den Etikettenabschnitt 11 vollständig vom Überziehabschnitt zu separieren. Der Nachteil ist dabei jedoch, dass dann das fertige Indikatoretikett unter Umständen nur schlecht von der Abziehschicht 5 abzulösen ist, nämlich dann, wenn die Abziehschicht 5 vergleichsweise gut an der Haftschicht 4 anhaftet und/oder vergleichsweise dünn und flexibel ist. Auch in den Ausführungsformen des Verfahrens, bei dem das Aufdrucken zuerst erfolgt, ist es daher vorteilhaft, anschließend nur die Trägerschicht 3 und die Haftschicht zu unterteilen.

Der Indikatorstreifen wird als Tinte in Form einer flüssigen Suspension, Dispersion oder Lösung der Indikatorsubstanz und gegebenenfalls weiterer Substanzen, etwa hydrophober und/oder filmbildender Substanzen, auf die Oberseite der Trägerschicht 3 aufgedruckt. Das Druckverfahren spielt im Rahmen vorliegender Erfindung keine Rolle. Das Trocknen des oder der Lösungsmittel dieser Tinte geschieht unweigerlich an den Rändern schneller als in der Mitte des Streifens 2. Daher kommt es zur Anreicherung von Indikatorsubstanz und anderen Feststoffen am Rand des Streifens und dort zur Ausbildung der in der **FIG. 3D** gezeigten Verdickungen in den gegenüberliegenden Endbereichen 2a und 2b des Indikatorstreifens 2. Der Überstand der verdickten Endbereiche 2a, 2b über den Rand des Etikettenabschnitts 11 ist nun vorteilhaft so gewählt, dass der direkt auf dem Etikettenbereich liegende Teil des Indikatorstreifens 2 eine möglichst gleichmäßige Dicke aufweist. Die verbleibende Dickenvariation des Indikatorstreifens oberhalb des Etikettenbereichs beträgt insbesondere bevorzugt 10% oder weniger, noch mehr bevorzugt 1% oder weniger.

Aus einem in dieser Weise behandelten Rohling 1 kann in einem letzten, in **Figur 3E** illustrierten Schritt durch Entfernen, beispielsweise Abziehen, des Überschussabschnittes 12 samt den auf diesem befindlichen verdickten Indikatorstreifenendbereichen 2a, 2b ein erfindungsgemäßes Indikatoretikett 1 fertiggestellt werden. Um zu vermeiden, dass bei dem Entfernen des Überschussbereichs 12 und der verdickten Enden, der oberhalb des Etikettenabschnitts liegende Teil des Indikatorstreifens beschädigt wird, werden Ausführungsformen des Verfahrens bevorzugt, bei denen zuerst der Indikatorstrei fen aufgedruckt wird und erst im Anschluss die Unterteilung des Etikettenrohlings 1' in Etikettenabschnitt 11 und Überschussabschnitt 12 erfolgt. Die Unterteilung wird vorteilhaft durch Stanzen oder Schneiden, ob mechanisch oder per Laser, vorgenommen.

Die **Figur 4** zeigt in schematischer Perspektive eine beispielhafte Ausführungsform eines Indikatoretikettenbandes, welches beispielsweise in einer Verpackungsmaschine wie der in Figur 7 gezeigten und untenstehend näher beschriebenen eingesetzt werden kann. Wie konkret dargestellt, trägt das Band 10 auf einer Oberseite einer bandförmigen Abziehschicht 50, im Folgenden auch als Abziehband 50 bezeichnet, eine Vielzahl hintereinander angeordneter Indikatoretiketten gemäß der zweiten Ausführungsform in der Variante mit kreisrundem Umriss der Figuren 2A, 2B und 3E entsprechen. Im Rahmen vorliegender Erfindung ist es aber ebenfalls möglich, dass das Abziehband 50 andere Ausführungsformen und/oder Varianten trägt. Es können auch auf demselben Band 10, 50 verschiedene Ausführungsformen und/oder Varianten gemischt vorkommen. Wie in der Figur dargestellt, sind die Indikatoretiketten 1 auf dem Abziehband 50 derart orientiert, dass der Indikatorstreifen 2 mit seiner lange Seite parallel zur Längsrichtung des Bandes 10, 50 ausgerichtet ist. Im Rahmen vorliegender Erfindung sind jedoch auch andere Ausrichtungen möglich, beispielsweise mit der langen Indikatorstreifenseite senkrecht zur Längsrichtung des Bandes 10, 50.

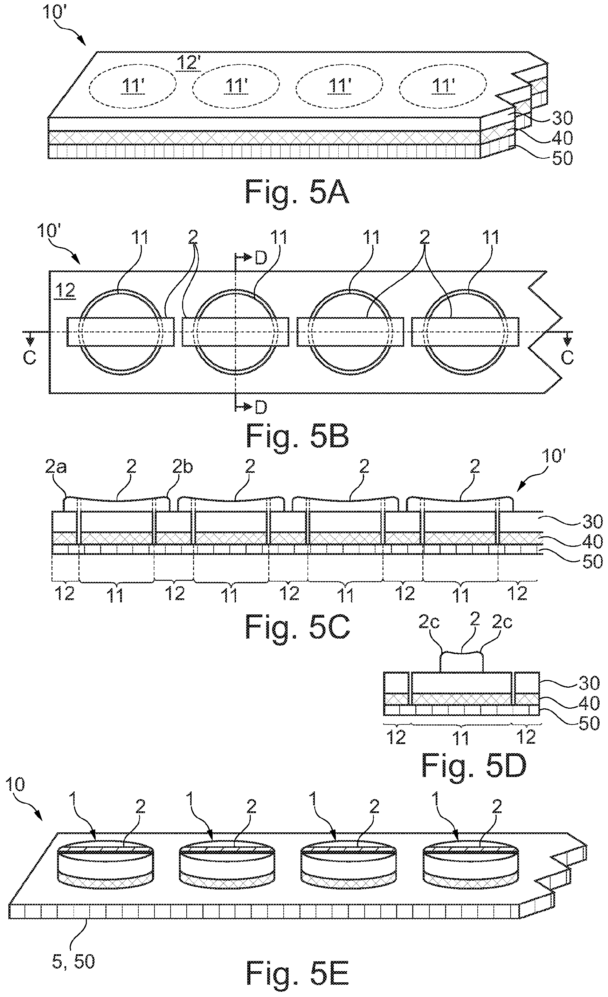

Die **Figuren 5A - 5E** illustrieren in mehreren schematischen Ansichten eine Ausführungsform einer Herstellung des Indikatoretikettenbandes 10 aus der vorangehenden Figur 4.

In der **Figur 5A** ist eine perspektivische Ansicht eines vorderen Abschnittes des Bandrohlings 10' gezeigt, auf dem mit gestrichelten Linien vier abzuteilende Etikettenabschnitte 11' angedeutet sind, welche von einem Überschussabschnitt 12' umgeben sind. Der Bandrohling 10' umfasst die drei in dieser Reihenfolge übereinander angeordneten, kongruenten bandförmigen Schichten Abziehschicht 50, Haftschicht 40 und Trägerschicht 30.

Die **Figur 5B** zeigt eine schematische Draufsicht auf den Figur 5A dargestellten Anfangsabschnitt nach dem Aufteilen zumindest der Trägerschicht 30, bevorzugt der Trägerschicht 30 und der Haftschicht 40 in Etikettenabschnitte 11 und einen Überschussabschnitt 12 sowie Aufdrucken jeweils eines Indikatorstreifens 2 pro Etikettenabschnitt. Der Überschussabschnitt 12 kann zu leichteren späteren Ablösung von der Abziehschicht auch in mehrere Unterabschnitte unterteilt sein, beispielsweise einen Unterabschnitt pro Etikettenabschnitt 11. Die Reihenfolge der beiden Schritte Aufteilen und Aufdrucken ist grundsätzlich beliebig, d.h. in manchen Ausführungsformen des Herstellungsverfahrens erfolgt das Aufteilen vor dem Aufdrucken, in anderen Ausführungsformen umgekehrt. Ersteres bietet den Vorteil, dass der Drucckopf für Indikatorsubstanzstreifen 2 anhand eines optisch erfassten Umrisses der Trennlinie/-spalte zwischen Etikettenabschnitt 11 und Überschussabschnitt/en 12 so positioniert werden kann, dass der Indikatorsubstanzstreifen möglichst genau mittig auf dem Etikettenabschnitt aufgedruckt wird. Die letztere Reihenfolge hat den Vorteil, dass das Entfernen, beispielsweise Abziehen, des Überschussabschnitts im letzten Schritt sauberer funktioniert, insbesondere der Indikatorstreifen nicht oder weniger stark beschädigt wird.

In den **Figuren 5C** und **5D** sind respektive ein Längsschnitt entlang der Linie C-C und ein Querschnitt entlang der Linie D-D der Figur 5B gezeigt. Wie in der Figur 5C nicht maßstabsgetreu angedeutet, weisen Indikatorstreifen 2 nach dem Trocknen verdickte Endbereiche 2a, 2b auf. Die Länge der gedruckten Streifen 2 ist nun in bevorzugten Ausführungsformen des Herstellungsverfahrens bewusst so gewählt, das diese Endbereich 2a, 2b oberhalb des Überschussbereichs 12 zu liegen kommen und somit zusammen mit diesem im letzten Schritt entfernt werden. Dann bleibt das in **Figur 5E** illustrierte fertige Indikatoretikettenband 10 zurück, auf dessen Abziehband 50 wie dargestellt die Indikatoretiketten 1 aufgereiht sind. Wie vorstehend beschrieben kann zuerst die Aufteilung in Etikettenabschnitte 11 und Überschussabschnitt/e 12 erfolgen oder umgekehrt. Im ersteren Fall bildet jeder Indikatorstreifen 2 nach dem Trocknen einen zusammenhängenden Block, im zweiten Fall hingegen sind die Endbereiche 2a, 2b von dem über dem Etikettenabschnitt befindlichen Mittelbereich durch einen im Zuge der Aufteilung/- trennung, welche beispielsweise mit einer Stanze erzeugt werden kann, voneinander getrennt. Diese beiden

Varianten werden in der Figur 5C durch die gestrichelten Linien zwischen den Mittelbereichen und den Endbereichen 2a, 2b angedeutet.

Der in der **Figur 5D** gezeigte Querschnitt verdeutlicht die laterale Einteilung der Trägerschicht 30 und der Haftschicht 40 sowie den Dickenverlauf des Indikatorstreifens 2 in der lateralen Richtung. Ebenso wie in longitudinaler Richtung, stellt sich beim Trocknen der aufgedruckten Indikatorsubstanztinte ein Profil mit verdickten Randbereichen 2c ein. Diese Verdickung stellt aber bei der optischen Vermessung eines Indikatoretiketts 1 kein Problem dar, wenn darauf geachtet wird, dass eine Relativbewegung zwischen optischem Sensor und Indikatoretikett 1 im Wesentlichen nur parallel zur Längsrichtung des Indikatorstreifen 2 erfolgt, weil dann der vom optischen Sensor erfasste Streifenquerschnitt sich nicht ändert und somit keine ungewollte zeitliche Variation des erfassten Signals gegeben ist.

In den Figuren ist ein Band 10 mit einer Reihe an hintereinander aufgereihten Indikatoretiketten 1 gezeigt. Im Rahmen der Erfindung ist es jedoch genauso denkbar, ein breiteres Band mit zwei, drei oder mehr Reihen von Indikatoretiketten herzustellen. Die Indikatoretiketten können hierbei auf einem quadratischen, dreiecks- oder anderen Gitter oder auch ohne konkrete regelmäßige Ordnung angeordnet sein.

Die **Figur 6** zeigt schematisch einen Längsschnitt durch eine erfindungsgemäße Verpackung mit einem an der Innenseite angehefteten Indikatoretikett. Die Verpackung 6 besteht in bekannter Weise aus einem schalenförmigen Unterteil 61, welches durch eine Oberseite dünne Folie oder einen Film 62 bedeckt und mittels der am Rand von Unterteil 61 und Folie 62 vorgenommenen Versiegelung 63 gasdicht verschlossen ist.

Im Inneren der Verpackung findet sich die Ware 7, etwa ein Lebensmittel, sowie, nicht dargestellt eine modifizierte Atmosphäre, also eine Atmosphäre mit einer anderer Zusammensetzung als die sonstige Erdatmosphäre, welche näherungsweise aus 79% Stickstoff und 21% Sauerstoff plus einiger Spurengase, vor allem Kohlendioxid, besteht. Beispielsweise kann in der Verpackung zur Verlangsamung der Alterung der Ware 7, also Erhöhung ihrer Lebensdauer oder (Mindest-)Haltbarkeit, eine sauerstofffreie Inertgasatmosphäre aus Stickstoff und/oder Edelgasen vorhanden sein. Das Ziel wäre hier ein Sauerstoffanteil von idealerweise 0%. Mathematisch exakt ist dies praktisch sowieso nicht erreichbar, besonders bei durch industrielle Massenfertigungsprozessen hergestellten Verpackungen wird dieses Ziel jedoch recht deutlich verfehlt und Restsauerstoffgehalte im Bereich von 0,5% bis 1% sind üblich. Bereits eine solche Niedrigsauerstoffatmosphäre bewirkt aber eine sehr deutliche Haltbarkeitssteigerung bei Lebensmitteln.

Der konkret in einer Verpackung 6 vorhandene Restsauerstoffgehalt kann nun mittels optischem Auslesen des im Inneren mit Kontakt zur Atmosphäre angebrachten, also angehefteten, Indikatoretiketts mit einer für Sauerstoff sensitiven Indikatorsubstanz gemessen werden. Hierzu ist das Etikett in einem transparenten Bereich 620 der oberseitigen Folie 62 angeordnet. Hierbei bezieht sich "transparent" auf die für die Messung relevanten Lichtfrequenzen, nicht notwendig auf eine Transparenz für sichtbares Licht.

Die **Figur 7** illustriert schematisch eine Verpackungsmaschine zur Herstellung von Verpackungen wie der in Figur 6 gezeigten, wofür die Maschine 100 unter anderem über Etikettiermittel zum Anbringen von Indikatoretiketten 1 verfügt. Beispielhaft sind diese hier als zwei jeweils um die z- und y-Achse verschwenkbare Arme 120 ausgebildet, auf welche jeweils eine zusammengerolltes Indikatoretikettenband 10 aufgesteckt ist, das durch eine im Bereich des distalen Endes 121 des Armes 120 befindliche Umlenkrolle 122 abgerollt wird. Durch die beim Umlenken entstehende Krümmung des Bandes 10 löst sich das jeweils an der Spitze 121 befindliche Etikett an einem unteren Rand ab. Durch die Anordnung der das Folienband 162 mit einer Geschwindigkeit V in die Richtung der Pfeile transportierenden Umlenkrollen 101 wird die spätere Innenseite der Folie 162 bis kurz vor das distale Ende der Etikettierarme 120 befördert, so dass die sich ablösenden Etiketten auf die Innenseite geklebt werden können.

Die optischen Erfassungsmittel 130, beispielsweise ein Kamerasystem, erfassen falls nötig transparente Bereiche der Folie 162, innerhalb der die Indikatoretiketten 1 angebracht werden sollen bzw. müssen, um nach dem Verschließen und Versiegeln der Verpackungen von außen optisch erfassbar zu sein. Die optischen Erfassungsmittel 130 sind dann nützlich, wenn die Folie 162, für das bei der optischen Messung der Indikatoretiketten verwendete Licht undurchsichtige Bereiche aufweist, etwa weil die Folie teilweise bedruckt oder mit Metall bedampft ist oder abschnittsweise aus verschiedenen Materialien besteht, etwa als Kombination aus einem Metall- und einem Kunststofffilm, und diese Bereiche nicht immer an derselben Stelle sind, entweder aufgrund von Fertigungstoleranzen oder weil ein einem Verpackungsdurchlauf Folien mit bewusst unterschiedlicher Gestaltung verwendet werden.

Dann kann es dazu kommen, dass die für die Indikatoretiketten vorgesehenen und verwendenden transparenten Bereiche, hier beispielhaft als in zwei parallelen Reihen in der Folie 162 vorhandene rechteckige Bereiche 1620 illustriert, im Verlauf der Folie 162 in transversaler wie longitudinaler Richtung nicht immer genau an derselben Stelle liegen. In manchen Ausführungsformen der Verpackungsmaschine werden longitudinale Abweichungen durch eine Steuerung der Abrollgeschwindigkeit der Indikatoretikettenbänder 10 auf den jeweiligen Armen 120 ausgeglichen, wobei die Geschwindigkeit erhöht wird, wenn die optischen Erfassungsmittel einen niedrigeren Abstand zwischen aufeinander folgenden transparenten Bereichen melden und umgekehrt, verlangsamt wird bei einem Abstand, der höher ist. Sind die Etikettierarme 120 wie angedeutet jeweils auch um die y-Achse y1 bzw. y2 verschwenkbar, können longitudinale Abweichungen auch alternativ oder zusätzlich hierdurch ausgeglichen werden.

Transversale Abweichungen können hingegen nur durch eine Repositionierung der Armspitze 121 in transversale Richtung ausgeglichen werden. Dies kann wie in der Figur 7 angedeutet durch Verschenken um die jeweilige z-Achse z1 bzw. z2 erfolgen. Alternativ (oder zusätzlich) können die Arme 120 auch transversal, also in y-Richtung, verschiebbar montiert sein.

Mit anderen, nicht dargestellten, Mitteln der Verpackungsmaschine 100 werden Formate 161 von Unterteilen 61 für die herzustellenden Verpackungen mit der gleichen Geschwindigkeit V herantransportiert. Lediglich beispielhaft gezeigt ist in der Figur eine 3x2 Anordnung von drei Verpackungsunterschalen 61 in longitudinaler und zwei in transversaler Richtung. Alternative Anordnungen sind ebenfalls möglich. Die Folie 162 wird durch die Umlenkrollen 101 mit der Seite, auf welche von den Etikettierarmen 120 die Indikatoretiketten 1 in den transparenten Bereichen 1620 angebracht wurden nach unten gekehrt und durch die Siegelstation 110 auf die Formate 161 aufgesiegelt, wobei gleichzeitig in die Verpackung eine modifizierte Atmosphäre, etwa wie oben beschrieben eine Stickstoffatmosphäre, eingeleitet wird. Hierdurch befinden sich die Indikatoretiketten im Inneren der Verpackungen 6 und stehen mit der dortigen Atmosphäre in Kontakt.

Dadurch ist es möglich mittels der optischen Sensoren 180 die Anwesenheit oder Konzentration einer Gaskomponente dieser Atmosphäre, beispielsweise Sauerstoff, oder eines anderen Analyten, etwa Abbauprodukte wie Ammoniak oder Schwefelwasserstoffe, welche auf eine fortgeschrittene Alterung oder ein Verderben der eingepackten Ware hindeuten, zu messen. Eine einzelne Reihe an Sensoren, wie in Figur 7 dargestellt, ermöglicht bereits die Nachkontrolle der in den Verpackungen 160 geschaffenen modifizierten Atmosphäre. Sind für jede der Bahnen, hier dargestellt sind zwei parallele Bahnen, so dass n x 2-Verpackungsformate verarbeitbar sind, in Transportrichtung V hintereinander zwei optische Sensoren 180 vorhanden, kann nach dem Versiegeln eine Dichtigkeitsprüfung durchgeführt werden, bei der die Verpackungsformate 161 nach Ermittlung der Atmosphärenkomposition unmittelbar nach dem Versiegeln einem sich schnell ändernden externen Atmosphärendruck ausgesetzt sind und anschließen eine Änderung der Zusammensetzung der Atmosphäre in den Verpackungen gemessen wird, etwa eine Erhöhung des Sauerstoffgehalts. Verpackungen, bei denen eine deutliche Änderung festgestellt wird, können nach dem Vereinzeln (mechanischen Trennen der einzelnen Verpackungen 6 eines Formates 161) ausgesondert oder alternativ, etwa wenn die Undichtigkeit nicht sehr gravierend ist, mit einem individuellen, üblicherweise reduzierten Haltbarkeitsdatum versehen werden.

### Bezugszeichenliste

- 1: Indikatoretikett
- 1': Indikatoretikettenrohling
- 2: Indikatorsubstanzpunkt/-streifen/-schicht
- 2a, 2b: stirnseitige Verdickung
- 2c: längsseitige Verdickung
- 3: Trägerschicht von 1, 1'
- 4: Haftschicht von 1, 1'
- 5: Abziehschicht
- 10: Indikatoretikettenband
- 10': Indikatoretikettenbandrohling
- 11: Etikettenbereich
- 12: Überschussbereich
- 30: bandförmige Trägerschicht von 10, 10'
- 40: bandförmige Haftschicht von 10, 10'
- 50: Abziehschicht von 10, 10'
- 6: Verpackung
- 61: Unterteil
- 62: Deckfolie
- 620: transparenter Bereich in 62
- 63: Siegelrand, 61 und 62 verbindend
- 7: Verpackungsinhalt
- 8: optischer Sensor
- L: Lichtfeld
- 100: Verpackungsmaschine
- 110: Siegelstation
- 120: Anbringarme
- 121: distales Ende von 120
- 122: Umlenkrolle
- 130: optische Erfassungsmittel
- V: Geschwindigkeit
- y1, y2: y-/Nick-Achse von 120
- z1, z2: z-/Gier-Achse von 120
- 160: versiegelte Verpackung
- 161: Format aus mehreren, rasterförmig angeordneten Unterteilen
- 162: Deckfolie
- 1620: transparente Bereiche in 162
- 180: optischer Sensor zum Erfassen und Ausmessen von 1

## Patentansprüche

1. Verpackung für ein Lebensmittel oder ein anderes Verpackungsgut, wobei die Verpackung (6) umfasst:
- ein flächiges oder schalenförmiges Unterteil (61), welches durch eine zumindest teilweise für Anregungs- und Fluoreszenzlicht transparente Folie (62) geschlossen ist, und
- ein Indikatoretikett (1) zur optischen Bestimmung eines Analyten einer in der Verpackung (6) vorhandenen Atmossphäre, wobei das Indikatoretikett umfasst:
o eine Trägerschicht (3),
o eine auf einer Unterseite der Trägerschicht (3) flächig aufgebrachte Haftschicht (4), und
o einen Indikatorsubstanzpunkt (2) auf einer der Unterseite gegenüberliegenden Oberseite der Trägerschicht (3), wobei der Indikatorsubstanzpunkt (2) eine gleichförmige Dicke, also Erstreckung in die zu der Oberseite der Trägerschicht (3) senkrechte Richtung, aufweist und eine Indikatorsubstanz enthält,
**dadurch gekennzeichnet dass,**
• die Indikatorsubstanz die optische Bestimmung der Anwesenheit und/oder der Konzentration des Analyten mittels Anregung der Indikatorsubstanz mit Anregungslicht und Auslesen von der Indikatorsubstanz ausgesendetem Fluoreszenzlicht erlaubt, und
• das Indikatoretikett (1) in einem für Anregungs- und Fluoreszenzlicht transparenten Bereich (620) der Folie (62) an die mit der Atmosphäre in der Verpackung (6) in Kontakt stehende Innenseite der Folie (62) geklebt ist.

2. Verpackung nach Anspruch 1, wobei das Indikatoretikett (1) für sichtbares Licht im Wesentlichen transparent, bevorzugt auch farblos ist.

3. Verpackung nach Anspruch 1 oder 2, wobei der Indikatorsubstanzpunkt (2) eine relative Dickenabweichung von 10% oder weniger, oder 1% oder weniger, aufweist.

4. Verpackung nach einem der vorhergehenden Ansprüche, wobei der Indikatorsubstanzpunkt (2)
- länglich ist und ein Länge-zu-Breite-Verhältnis von zwischen 2 und 20, bevorzugt zwischen 5 und 10 aufweist, und/oder
- ein in etwa rechteckiger Streifen zwischen 0,5 und 2 Millimeter, bevorzugt 1 Millimetern, Breite und zwischen 4 und 12 Millimeter, bevorzugt 7 oder 8 Millimeter Länge ist.

5. Verpackung nach einem der vorhergehenden Ansprüche, wobei sie auch an der Stelle (620), an der das Indikatoretikett (1) aufgeklebt ist, einen Aufdruck aufweist, der jedoch für Anregungs- und Fluoreszenzlicht ausreichend transparent ist.

6. Verpackung nach dem vorhergehenden Anspruch, wobei für das Herstellen des Aufdrucks eine für das Anregungs- und Fluoreszenzlicht transparente Tinte verwendet ist.

7. Verpackung nach einem der beiden vorhergehenden Ansprüche, wobei der Aufdruck im Bereich einer von dem Indikatoretikett (1) beanspruchten Fläche bedruckte und nicht bedruckte Bereiche aufweist und die nichtbedruckten Bereiche mindestens 5%, odermindestens 10%, oder mindestens 25% der Fläche des Indikatoretiketts ausmachen.

8. Verpackung nach dem vorhergehenden Anspruch, wobei der Aufdruck ein mittels Digitaldruck aufgebrachtes Raster von Rasterpunkten ist und ein Abstand zwischen benachbarten Punkten des Rasters dem 1,05 - 1,5 fachen eines Radius der Rasterpunkte ist.

## Claims

1. Packaging for a foodstuff or another good, wherein the packaging (6) comprises:
- a flat or dish-shaped lower part (61) which is closed by a film (62) which is at least partially transparent to excitation and fluorescent light, and
- an indicator label (1) for the optical determination of an analyte in an atmossphere present inside the packaging (6), wherein the indicator label comprises:
∘ a carrier layer (3)
∘ an adhesive layer (4) applied to the underside of the carrier layer (3) over its area, and
∘ an indicator substance dot (2) on the upper side of the carrier layer (3) opposite the underside, wherein the indicator substance dot (2) has a uniform thickness, i.e. extension in the direction perpendicular to the upper side of the carrier layer (3) and contains an indicator substance
**characterized in that**
• the indicator substance allows the optical determination of the presence and/or concentration of the analyte by means of excitation of the indicator substance with excitation light and reading out the fluorescence light emitted by the indicator substance, and
• the indicator label (1) is adhered in a region (620) of the film which is transparent to excitation and fluorescence light to the inner side of the film (62) which is in contact with the atmosphere in the packaging.

2. Packaging according to claim 1, wherein the indicator label (1) is substantially transparent for visible light, and preferably also colorless.

3. Packaging according to claim1 or 2, wherein the indicator substance dot (2) has a relative thickness deviation of 10% or less or 1% or less.

4. Packaging according to one of the preceding claims, wherein the indicator substance dot (2)
- is elongated and has a length-to-width-ratio of between 2 and 20, preferably between 5 and 10, and/or
- is an approximately rectangular strip between 0,5 and 2 Millimeters , preferably 1 Millimeter, in width and between 4 and 12 Millimeters, preferably 7 or 8 Millimeters, in length.

5. Packaging according to one of the preceding claims, wherein it also has an imprint at the location (620) where the indicator label (1) is adhered, but which imprint is sufficiently transparent for excitation and fluorescent light.

6. Packaging according to the preceding claim, wherein an ink transparent to the excitation and fluorescent light is used for producing the imprint.

7. Packaging according to one of the two preceding claims, wherein in the area covered by the indicator label (1) the imprint has printed and non-printed areas wherein the non-printed areas make up at least 5%, or at least 10%, or at least 25% of the area of the indicator label.

8. Packaging according to the preceding claim, wherein the imprint is a grid of halftone dots applied by digital printing and a distance between neighbouring dots of the grid is 1.05 - 1.5 times a radius of the halftone dots.

## Revendications

1. Emballage pour une denrée alimentaire ou un autre produit, l'emballage (6) comprenant :
- une partie inférieure (61) plate ou en forme de cuvette, fermée par un film (62) au moins partiellement transparent à la lumière de l'excitation et de fluorescence, et
- une étiquette indicatrice (1) pour la détermination optique d'un analyte dans l'atmosphère présente à l'intérieur de l'emballage (6), l'étiquette indicatrice comprenant:
∘ une couche support (3)
∘ une couche adhésive (4) appliquée sur la face inférieure de la couche support (3) sur toute sa surface, et
∘ un point de substance indicatrice (2) sur la face supérieure de la couche support (3), opposée à la face inférieure, le point de substance indicatrice (2) présentant une épaisseur uniforme, c'est-à-dire une extension perpendiculaire à la face supérieure de la couche support (3), et contienant une substance indicatrice,
**caractérisé en ce que**
• la substance indicatrice permet la détermination optique de la présence et/ou de la concentration de l'analyte par excitation de la substance indicatrice avec lumière d'excitation et lecture de la lumière de fluorescence émise par la substance indicatrice,
• l'étiquette indicatrice (1) etant collée dans une zone (620) du film transparente à la lumière d'excitation et de fluorescence, sur la face interne du film (62) en contact avec l'atmosphère de l'emballage.

2. Emballage selon la revendication 1, dans lequel l'étiquette indicatrice (1) est essentiellement transparente à la lumière visible, et de préférence est également incolore.

3. Emballage selon la revendication 1 ou 2, dans lequel le point de substance indicatrice (2) présente une écart d'épaisseur relative de 10% ou moins ou 1% ou moins.

4. Emballage selon l'une des revendications précédentes, dans lequel le point de substance indicatrice (2)
- est allongé et présente un rapport longueur/largeur compris entre 2 et 20, de préférence entre 5 et 10, et/ou
- est une bande approximativement rectangulaire de 0,5 à 2 millimètres, de préférence 1 millimètre, de largeur et de 4 à 12 millimètres, de préférence 7 ou 8 millimètres, de longueur.

5. Emballage selon l'une des revendication précédantes, dans lequel il comporte egalement une impression à l'endroit (620) où est collée l'étiquette indicatrice (1), mais cette impression est suffisamment transparente à la lumière d'excitation et la lumière de fluorescence.

6. Emballage selon la revendication précédante, dans lequel une encre transparante à la lumière d'excitation et la lumière de fluorescence est utilisée pour realiser l'impression.

7. Emballage selon l'une des deux revendications précédentes, dans lequel, dans la zone couverte par l'étiquette indicatrice (1), l'impression présente des zones imprimées et non imprimées, les zones non imprimées constituant au moins 5 %, ou au moins 10 %, ou au moins 25 % de la surface de l'étiquette indicatrice.

8. Emballage selon la revendication précédante, dans lequel l'impression est une grille de points en demi-teintes appliquée par impression numérique et une distance entre les points voisins de la grille est de 1,05 à 1,5 fois la rayon des points en demi-teintes.
